(19) Europäisches Patentamt
European Patent Office
Office européen des brevets

(11) EP 0 815 828 B1

(12) FASCICULE DE BREVET EUROPEEN

(45) Date de publication et mention de la délivrance du brevet: **24.02.1999 Bulletin 1999/08**

(51) Int. Cl.[6]: **A61K 7/06**, A61K 7/48

(21) Numéro de dépôt: **97401400.3**

(22) Date de dépôt: **18.06.1997**

# (54) Utilisation en cosmétique d'un poly(acide 2-acrylamido 2-méthyl propane sulfonique) réticulé et neutralisé à au moins 90% et compositions topiques les contenant

Kosmetische Verwendung und Präparate einer verzweigten und mindestens auf 90% neutralisierten Polyacrylamidomethylpropansulfonsäure

Cosmetic use of and composition containing a crosslinked and at least 90% neutralized polyacrylamidoemthylpropane sulfonic acid

(84) Etats contractants désignés:
**AT BE CH DE ES FR GB IT LI NL PT**

(30) Priorité: **28.06.1996 FR 9608107**

(43) Date de publication de la demande:
**07.01.1998 Bulletin 1998/02**

(73) Titulaire: **L'OREAL**
**75008 Paris (FR)**

(72) Inventeurs:
- **Dupuis, Christine**
  **75018 Paris (FR)**
- **Hansenne, Isabelle**
  **75017 Paris (FR)**
- **Maubru, Mireille**
  **78400 Chatou (FR)**
- **Sebillotte-Arnaud, Laurence**
  **94240 L'Hay Les Roses (FR)**
- **Lorant, Raluca**
  **94320 Thiais (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**EP-A- 0 466 184**
**FR-A- 2 681 245**
**FR-A- 2 698 004**

EP 0 815 828 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente demande concerne l'utilisation comme produit cosmétique de polymères réticulés poly(acide 2-acrylamido 2-méthylpropane sulfonique) neutralisés à au moins 90% et les compositions cosmétiques ou dermatologiques le contenant

Les compositions cosmétiques ou dermatologiques présentent en général une viscosité élevée et sont pour la plupart formulées sous une forme liquide épaissie telle qu'un lait, une crème, un gel ou une pâte. Ce type de présentation est très appréciée par le consommateur ; il correspond le plus souvent à une préoccupation pratique pour le formulateur : faciliter la prise du produit hors de son conditionnement sans perte significative, limiter la diffusion du produit à la zone locale de traitement et pouvoir l'utiliser dans des quantités suffisantes pour obtenir l'effet cosmétique ou dermatologique recherché.

Cet objectif est important pour les formulations telles que celles des produits pour le soin, l'hygiène ou le maquillage qui doivent bien s'étaler de façon homogène sur la surface locale à traiter ainsi que les compositions capillaires qui doivent bien s'étaler et se répartir de façon régulière le long des fibres kératiniques et ne pas ruisseler sur le front, la nuque ou le visage ou dans les yeux.

Pour satisfaire à ces conditions, on augmente la viscosité des compositions par l'ajout de polymères épaississants et/ou gélifiants.

On connaît dans l'état de la technique des homopolymères poly(acide 2-acrylamido 2-méthylpropane sulfonique) comme les produits commerciaux COSMEDIA HSP1160 et RHEOTIK 8011 de la société HENKEL. Ils sont utilisés comme agents épaississants et/ou gélifiants dans de nombreuses formulations cosmétiques. Ces polymères ne permettent pas d'obtenir des compositions stables et homogènes pouvant atteindre des viscosités élevées dans une large gamme de pH. Ils conduisent le plus souvent à des gels fluides, hétérogènes, filants et collants.

La demanderesse a découvert de manière surprenante une nouvelle famille de polymères épaississants et/ou gélifiants permettant d'obtenir un très grand nombre de formulations cosmétiques et dermatologiques pouvant contenir des supports de nature différente.

L'invention a pour objet l'utilisation comme épaississant et/ou gélifiant dans des compositions cosmétiques et/ou dermatologiques de poly(acide 2-acrylamido 2-méthylpropane sulfonique) réticulés et neutralisés à au moins 90% et que l'on définiera plus en détail dans la suite de la description.

Ces polymères permettent notamment de préparer de nombreuses compositions aqueuses, dans une large gamme de pH, dont la viscosité reste stable dans le temps à température ambiante ou à des températures plus élevées.

Ils permettent en outre de réaliser des gels aqueux transparents, homogènes, non-coulants, non-filants, doux et glissants à l'application et stables à la conservation.

Les polymères poly(acide 2-acrylamido 2-méthylpropane sulfonique) réticulés et pratiquement ou totalement neutralisés, conformes à l'invention, sont hydrosolubles ou gonflables dans l'eau. Ils sont en général caractérisés par le fait qu'ils comprennent, distribués de façon aléatoire :

a) de 90 à 99,9% en poids de motifs de formule générale (1) suivante :

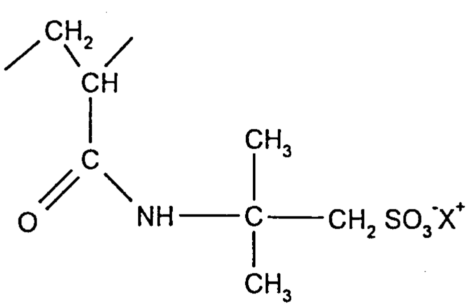

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

De façon préférentielle, les polymères de l'invention comportent un nombre de motifs de formule (1) dans une quantité suffisamment élevée pour obtenir des particules de polymère dont le volume hydrodynamique en solution d'eau présente un rayon allant de 10 à 500 nm et dont la distribution est homogène et unimodale.

Les polymères selon l'invention plus particulièrement préférés comprennent de 98 à 99,5 % en poids de motifs de formule (1) et de 0,2 à 2 % en poids de motifs réticulants.

$X^+$ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations $NH_4^+$ et 0 à 10% mole sont des protons ($H^+$).

Les monomères de réticulation ayant au moins deux doubles-liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylène-glycol, la triallylamine, le triméthylolpropane-diallyléther, le méthylen-bis-acrylamide ou le divinylbenzène.

Les monomères de réticulation ayant au moins deux doubles-liaisons oléfiniques sont plus particulièrement choisis parmi ceux répondant à la formule générale (2) suivante :

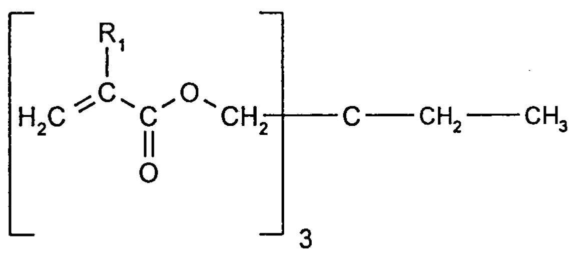 (2)

dans laquelle $R_1$ désigne un atome d'hydrogène ou un alkyle en $C_1$-$C_4$ et plus particulièrement méthyle (triméthylol propane triacrylate).

La réaction de polymérisation des polymères de l'invention produit non seulement des chaînes linéaires mais aussi des molécules de polymère ramifiées ou réticulées. Ces molécules peuvent être caractérisées notamment par leur comportement rhéologique dans l'eau mais plus particulièrement par la diffusion de la lumière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec:

- M désignant la masse en grammes de la macromolécule non-dissoute ;
- $N_A$ désignant le nombre d'Avogadro ;
- $V_1$ désignant le volume spécifique du solvant ;
- $V_2$ désignant le volume spécifique de la macromolécule ;
- d la masse en grammes du solvant qui est associé avec 1 gramme de macromolécule non-dissoute.

Si la particule hydrodynamique est sphérique, il est alors facile de calculer à partir du volume hydrodynamique le rayon hydrodynamique par la formule :

$$v_h = 4\Pi R^3/3$$

avec R désignant le rayon dynamique.

Les cas où les particules hydrodynamiques sont des sphères parfaites sont extrêmement rares. La majorité des polymères synthétiques impliquent des structures compactées ou des ellipsoides à haute excentricité. Dans ce

cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution , on en déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990 ;
(4) Provincher S.W. ; Comp. Phys., 27, 213, 1982 ;
(5) Provincher S.W. ; Comp. Phys., 27, 229, 1982 ;
(6) ALV Laservertriebgesellschaft mbH, Robert Bosch Str. 47, D-63225 Langen, Germany ;
(7) ELS-Reinheimer Strasse 11, D-64846 Gross-Zimmern, Germany ;
(8) CHI WU et al, Macromolecules, 1995, 28,4914-4919.

Les polymères particulièrement préférés sont ceux présentant une viscosité mesurée au viscosimètre BROOKFIELD, mobile 4, à une vitesse de rotation de 100 tours/minutes dans une solution d'eau à 2 % et à 25 °C supérieure ou égale à 1000 mPas (cps) et plus préférentiellement allant de 5000 à 40000 cps et plus particulièrement de 6500 à 35000 mPas (cps).

Les poly(acide 2-acrylamido 2-méthylpropane sulfonique) réticulés de l'invention peuvent être obtenus selon le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol;
(b) on neutralise la solution ou la dispersion de monomère obtenue en (a) par une ou plusieurs bases minérales ou organiques, de préférence l'ammoniaque $NH_3$, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Un autre objet de l'invention consiste en des compositions cosmétiques ou dermatologiques contenant dans un milieu cosmétiquement acceptable au moins un poly(acide 2-acrylamido 2-méthylpropane sulfonique) réticulé et neutralisé à au moins 90% tel que décrit précédemment.

Les poly(acide 2-acrylamido 2-méthylpropane sulfonique) réticulés, pratiquement ou totalement neutralisés sont présents dans les compositions cosmétiques ou dermatologiques de l'invention dans des concentrations allant préférentiellement de 0,01 à 20% en poids par rapport au poids total de la composition et plus préférentiellement de 0,1 à 10% en poids.

Les compositions de l'invention contiennent un milieu cosmétiquement ou dermatologiquement acceptable, c'est-à-dire un milieu compatible avec toutes les matières kératiniques telles que la peau, les ongles, les muqueuses et les cheveux ou toute autre zone cutanée du corps.

Les compositions contiennent de préférence un milieu aqueux cosmétiquement et/ou dermatologiquement acceptable. Elles présentent un pH pouvant aller de préférence de 1 à 13 et plus préférentiellement de 2 à 12.

Le milieu cosmétiquement et/ou dermatologiquement acceptable des compositions selon l'invention est plus particulièrement constitué d'eau et éventuellement de solvants organiques cosmétiquement et/ou dermatologiquement acceptables.

Les solvants organiques peuvent représenter de 5 % à 98 % du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des mono-alcools inférieurs linéaires ou ramifiés ayant de 1 à 8 atomes de carbone comme l'éthanol, le propanol, le butanol, l'isopropanol, l'isobutanol ; des

polyéthylène glycols ayant de 6 à 80 oxydes d'éthylène ; des polyols tels que le propylène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer des polyols tels que des dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Afin que les compositions cosmétiques ou dermatologiques de l'invention soient plus agréables à utiliser (plus douce à l'application, plus nourrissante, plus émolliente), il est possible d'ajouter une phase grasse dans le milieu de ces compositions.

La phase grasse représente de préférence, de 0 % à 50 % du poids total de la composition.

Cette phase grasse peut comporter une ou plusieurs huiles choisies de préférence dans le groupe constitué par :

- les silicones volatiles ou non-volatiles, linéaires, ramifiées ou cycliques, organomodifiées ou non, hydrosolubles ou liposolubles,
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de purcellin, les isoparaffines,
- les huiles fluorées et perfluorées,
- les esters d'acides gras tels que l'huile de Purcellin.

Elle peut aussi comporter comme matière grasse un(e) ou plusieurs alcools gras, acides gras (acide stéarique) ou cires (paraffine, cires de polyéthylène, carnauba, cire d'abeilles).

De façon connue, toutes les compositions de l'invention peuvent contenir des adjuvants habituels dans les domaines cosmétique et dermatologique, d'autres gélifiants et/ou épaississants classiques ; des polymères ; des émulsionnants ; des tensio-actifs ; des agents hydratants ; des émollients ; des filtres solaires ; des actifs hydrophiles ou lipophiles comme des céramides ; des agents anti-radicaux libres ; des répulsifs pour insectes ; des agents amincissants ; des bactéricides ; des séquestrants ; des antipelliculaires ; des antioxydants ; des conservateurs ; des agents alcanisants ou acidifiants ; des parfums ; des charges ; des matières colorantes. Les quantités de ces différents adjuvants sont celles classiquement utilisées dans les domaines considérés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes appropriées pour une application topique, notamment sous forme de solutions du type lotion ou sérum ; sous forme de gels aqueux ; sous forme d'émulsions obtenues par dispersion d'une phase grasse dans une phase aqueuse (H/E) ou inversement (E/H), de consistance liquide, semi-liquide ou solide telles que des laits, des crèmes plus ou moins onctueuses, des pâtes. Ces compositions sont préparées selon les méthodes usuelles.

Les compositions selon l'invention peuvent être utilisées comme produits rincés ou comme produits non-rincés capillaires notamment pour le lavage, le soin, le conditionnement, le maintien de la coiffure ou la mise en forme des fibres kératiniques telles que les cheveux.

Elles peuvent être des produits de coiffage tels que des lotions de mise en plis, des lotions pour le brushing, des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une mousse pour la fixation ou le traitement des cheveux.
Les compositions de l'invention peuvent être également des shampooings, des compositions à rincer ou non, à appliquer avant ou après un shampooing, une coloration, une décoloration, une permanente ou un défrisage.

Les compositions de l'invention peuvent être également utilisées comme produit de soin et/ou l'hygiène tels que des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions de l'invention peuvent être également utilisées comme produit antisolaire.

Les compositions peuvent également consister en des préparations solides constituant des savons ou des pains de nettoyage.

Les compositions de l'invention peuvent être également utilisées comme produit de soin bucco-dentaire tel que des pâtes dentifrices, des bains de bouche.

Les compositions peuvent être des produits pour le maquillage tels que des crèmes pour le visage, des fonds de teint, des mascaras, des eye-liner, des rouges à lèvres.

Un autre objet de l'invention est un procédé de traitement non-thérapeutique cosmétique de la peau, du cuir chevelu, des cheveux, des cils, des sourcils, des ongles ou des muqueuses, caractérisé par le fait qu'on applique sur le support kératinique une composition telle que définie ci-dessus, selon la technique d'utilisation habituelle de cette composition. Par exemple : application de crèmes, de gels, de sérums, de lotions, de laits sur la peau, le cuir chevelu et/ou les muqueuses.

Les exemples suivants illustrent l'invention sans présenter un caractère limitatif.

## EXEMPLE DE PREPARATION A

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 32,0 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant viscosité mesurée au viscosimètre BROOKFIELD, mobile 4, à une vitesse de rotation de 100 tours/minutes dans une solution d'eau à 2 % et à 25 °C allant de 15000 mPas (cps) à 35.000 mPas (cps). La viscosité du polymère sera choisie et contrôlée selon des moyens classiques en fonction de l'application cosmétique envisagée.

Le rayon hydrodynamique du polymère obtenu dans une solution aqueuse déterminé par diffusion de la lumière dynamique est de 440 nm.

## EXEMPLE DE PREPARATION B

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 19,2 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant une viscosité mesurée au viscosimètre BROOKFIELD, mobile 4, à une vitesse de rotation de 100 tours/minutes dans une solution d'eau à 2 % et à 25 °C de l'ordre de 7000 mPas (cps).

Le rayon hydrodynamique du polymère obtenu dans une solution aqueuse détérminé par diffusion de la lumière dynamique est de 160 nm.

**EXEMPLE 1: Shampooing**

| | |
|---|---|
| - Lauryl éther sulfate de Sodium vendu sous le nom d'EMPICOL ESB3/FL par la Société ALBRIGHT ET WILSON | 10 g MA |
| - Poly(acide 2-acrylamido 2-méthylpropane sulfonique) réticulé et neutralisé par de l'ammoniaque préparé selon le procédé de l'exemple de préparation B de viscosité de l'ordre de 7.000 mPas (cps) dans une solution d'eau à 2% et à 25°C | 1,5 g MA |
| - Acide citrique | 3 g |
| - Eau pH ajusté à 4,8 (NaOH) qsp | 100 g |

Ce shampooing se présente sous l'aspect d'un liquide translucide épaissi. Il possède de bonnes propriétés moussantes.

**EXEMPLE 2 : Shampooing**

| | |
|---|---|
| - Lauryl éther sulfate de triéthanolamine | 5 g MA |
| - Lauryl éther sulfate de monoéthanolamine | 5 g MA |
| - Poly(acide 2-acrylamido 2-méthylpropane sulfonique) réticulé et neutralisé par de l'ammoniaque, préparé selon le procédé de l'exemple de préparation B de viscosité de l'ordre de 7.000 mPas (cps) dans une solution d'eau à 2% et à 25°C | 1,0 g MA |
| - Hydroxyéthylcellulose réticulée par l'épichlorhydrine et quaternisée par la triéthanolamine | 0,1 g |
| - Eau pH ajusté à 7,8 (NaOH) qsp | 100 g |

Ce shampooing se présente sous l'aspect d'un liquide translucide épaissi. Il possède de bonnes propriétés moussantes.

**EXEMPLE 3 : Gel de coiffage**

| | |
|---|---|
| - Copolymère vinylpyrrolidone/diméthylamino éthylméthacrylate vendu sous le nom COPOLYMER 845 par la société ISP | 1 g MA |
| - Poly(acide 2-acrylamido 2-méthylpropane sulfonique) réticulé et neutralisé par de l'ammoniaque préparé selon le procédé de l'exemple de préparation A de viscosité de l'ordre de 15.600 mPas (cps) dans une solution d'eau à 2% et à 25°C | 1,0 g MA |
| - Amino-2 méthyl-2 propanol-1 (AMP) pH ajusté à 7,5 qs | |
| - Ethanol absolu | 8,7 g |
| - Parfum, conservateur, colorant qs | |
| - Eau déminéralisée qsp | 100 g |

On obtient un gel stable, épais, transparent, onctueux et homogène.

**EXEMPLE 4 :** **Gel transparent solaire**

| | |
|---|---|
| - Glycérol | 4 g |
| - Poly(acide 2-acrylamido 2-méthylpropane sulfonique) réticulé et neutralisé par de l'ammoniaque préparé selon le procédé de l'exemple de préparation A de viscosité de l'ordre de 16.000 mPas (cps) dans une solution d'eau à 2% et à 25°C | 1,0 g MA |
| - Acide benzène-1,4-di(3-méthylidène-10-camphosulfonique)] en solution aqueuse à 33% | 6 g |
| - Propylèneglycol | 18 g |
| - Eau déminéralisée stérilisée | 70 g |
| pH = 1,7 | |

On obtient un gel stable, épais, transparent, onctueux et homogène.

**EXEMPLE 5 :** **Gel transparent anti-moustiques**

| | |
|---|---|
| - Glycérol | 4 g |
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 mPas (cps) dans une solution d'eau à 2% et à 25°C | 0,8 g MA |
| - N-butyl, N-acétyl aminopropionate d'éthyle | 15 g |
| - N,N diéthyl-M- toluamide | 20 g |
| - Propylèneglycol | 18 g |
| - Ethanol à 96° | 23 g |
| - Eau déminéralisée stérilisée | 19,2 g |
| pH = 3,95 | |

On obtient un gel stable, épais, transparent, onctueux et homogène.

**EXEMPLE 6 :** **Gel douche**

| | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 mPas (cps) dans une solution d'eau à 2% et à 25°C | 1,2 g MA |
| - Myristyl glycol de suif hydrogéné | 1 g |
| - Sel de sodium du p-hydroxybenzoate de méthyle | 0,215 g |
| - Sel disodique de l'acide éthylène diamine tétraacétique | 0,26 g |
| - Glycérol | 4 g |
| - Copolymère chlorure de diméthyl diallyl ammonium/acrylamide 50/50 en solution aqueuse à 8% | 0,5 g |
| - Diméthylol-1,3 diméthyl-5,5 hydantoïne en solution aqueuse de 55% | 0,172 g |

(suite)

| | |
|---|---|
| - Lauryl ether sulfate de sodium à 2,2 moles d'oxyde d'éthylène | 10 g |
| - Lauryl sulfate de triéthanolamine en solution aqueuse à 40% | 25 g |
| - Cocoylbétaïne en solution aqueuse à 32% | 5 g |
| - Parfum | 0,15 g |
| - NaOH qs pH 7,5 | |
| - Eau déminéralisée stérilisée qsp | 100 g |

On obtient un gel douche stable, transparent, onctueux et homogène et ayant de bonnes propriétés moussantes.

**EXEMPLE 7 :** **Gel douche**

| | |
|---|---|
| - Poly(acide 2-acrylamido 2-méthylpropane sulfonique) réticulé et neutralisé par de l'ammoniaque, préparé selon le procédé de l'exemple de préparation B de viscosité de l'ordre de 7.000 Mpas (cps) dans une solution d'eau à 2% et à 25°C | 1,2 g MA |
| - Myristyl glycol de suif hydrogéné | 1 g |
| - Sel de sodium du p-hydroxybenzoate de méthyle | 0,215 g |
| - Sel disodique de l'acide éthylène diamine tétraacétique | 0,26 g |
| - Glycérol | 4 g |
| - Copolymère chlorure de diméthyl diallyl ammonium/acrylamide 50/50 en solution aqueuse à 8% | 0,5 g |
| - Diméthylol-1,3 diméthyl-5,5 hydantoïne en solution aqueuse de 55% | 0,172 g |
| - Lauryl éther sulfate de sodium à 2,2 moles d'oxyde d'éthylène | 10 g |
| - Lauryl sulfate de triéthanolamine en solution aqueuse à 40% | 25 g |
| - Cocoylbétaïne en solution aqueuse à 32% | 5 g |
| - Parfum | 0,15 g |
| - HCl qs pH 5,5 | |
| - Eau déminéralisée stérilisée qsp | 100 g |

On obtient un gel douche stable, transparent, onctueux et homogène et ayant de bonnes propriétés moussantes.

**EXEMPLE 8 :** **Bain de bouche**

| | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 mPas (cps) dans une solution d'eau à 2% et à 25°C | 0,1 g MA |
| - P-hydroxybenzoate de méthyle | 0,1 g |
| - Glycérol | 5 g |
| - Mono-laurate de sorbitane oxyéthyléné à 20 moles d'oxyde d'éthylène | 0,4 g |

(suite)

| | |
|---|---|
| - Lauryl sulfate de sodium en poudre | 0,25 g |
| - Fluorure de sodium | 0,05 g |
| - Ethanol à 96° | 5 g |
| - Parfum | 18 g |
| - NaOH qs pH 7,5 | |
| - Eau déminéralisée stérilisée qsp | 100 g |

On obtient un liquide stable, transparent et homogène.

**EXEMPLE 9 : Bain de bouche**

| | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 mPas (cps) dans une solution d'eau à 2% et à 25°C | 0,1 g MA |
| - P-hydroxybenzoate de méthyle | 0,1 g |
| - Glycérol | 5 g |
| - Mono-laurate de sorbitane oxyéthylÉné à 20 moles d'oxyde d'éthylène | 0,4 g |
| - Lauryl sulfate de sodium en poudre | 0,25 g |
| - Fluorure de sodium | 0,05 g |
| - Ethanol à 96° | 5 g |
| - Parfum | 18 g |
| - HCl qs pH 5 | |
| - Eau déminéralisée stérilisée qsp | 100 g |

On obtient un liquide stable, transparent et homogène.

**EXEMPLE 10 : Gel rafraîchissant hydratant**

| | |
|---|---|
| - Poly(acide 2-acrylamido 2-méthylpropane sulfonique) réticulé et neutralisé par de l'ammoniaque, préparé selon le procédé de l'exemple de préparation B de viscosité de l'ordrede 7.000 mPas (cps) dans une solution d'eau à 2% et à 25°C | 1,5 g MA |
| - Glycérine | 3 g |
| - Alcool éthylique à 96° dans l'eau | 20 g |
| - Eau déminéralisée stérilisée qsp | 100 g |
| pH 4,8 | |

On obtient un gel transparent, stable et homogène.

**EXEMPLE 11 :** **Crème de soin (émulsion huile-dans-eau)**

*Phase grasse*

| | |
|---|---|
| - Monostéarate de sorbitan à 20 moles d'oxyde d'éthylène | 1 g |
| - Stéarate de glycérol | 2 g |
| - Acide stéarique | 1,4 g |
| - Triéthanolamine | 0,7 g |
| - Alcool cétylique | 0,5 g |
| - Huile de tournesol | 15 g |
| - Cyclométhicone | 5 g |

*Phase aqueuse*

| | |
|---|---|
| - Poly(acide 2-acrylamido 2-méthylpropane sulfonique) réticulé et neutralisé par de l'ammoniaque, préparé selon le procédé de l'exemple de préparation B de viscosité de l'ordre de 7.000 mPas (cps) dans une solution d'eau à 2% et à 25°C | 1,2 g MA |
| - Glycérine | 5 g |
| - Conservateur | 0,25 g |
| - Parfum | 0,15 g |
| - Eau déminéralisée stérilisée qsp | 100 g |
| pH 6,5 | |

On obtient une crème lisse, blanche et brillante.

**EXEMPLE 12 :** **Lait hydratant (émulsion huile-dans-eau)**

*Phase grasse*

| | |
|---|---|
| - Composition auto-émulsionnable vendue sous le nom MONTANOV 68 par la société SEPPIC | 2,5 g |
| - Huile de jojoba | 3 g |
| - Huile de maïs | 13 g |

*Phase aqueuse*

| | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 mPas (cps) dans une solution d'eau à 2% et à 25°C | 0,1 g MA |
| - Glycérine | 5 g |
| - Conservateur qs | |
| - Eau déminéralisée stérilisée qsp | 100 g |
| pH 6,0 | |

Ce lait s'applique facilement sur le visage et sur le corps et est plus particulièrement doux à l'application.

**<u>EXEMPLE 13</u> : Crème nettoyante visage et corps (émulsion huile-dans-eau)**

*Phase grasse*

| | |
|---|---|
| - Huile de jojoba | 20 g |

*Phase aqueuse*

| | |
|---|---|
| - Poly(acide 2-acrylamido 2-méthylpropane sulfonique) réticulé et eutralisé par de l'ammoniaque, préparé selon le procédé de l'exemple de préparation B de viscosité de l'ordre de 7.000 mPas (cps) dans une solution d'eau à 2 % et à 25°C | 2 g MA |
| - Alkyl($C_8$/$C_{10}C_{12}C_{14}$/$C_{16}$;29/37/23/9/2)polyglucoside-1,4 | 6 g |
| - Glycérine | 5 g |
| - Conservateur qs | |
| - Eau déminéralisée stérilisée qsp pH 5 | 100 g |

On obtient une crème lisse, onctueuse, brillante et moussante.

**<u>EXEMPLE 14</u> : Gel-crème contour des yeux (émulsion huile-dans-eau)**

*Phase grasse*

| | |
|---|---|
| - PEG-20 stéarate | 1,65 g |
| - Alcool cétylique | 1,05 g |
| - Glycéryl stéarate | 0,3 g |

(suite)

| | |
|---|---|
| - Cyclométhicone | 6 g |

*Phase aqueuse*

| | |
|---|---|
| - Poly(acide 2-acrylamido 2-méthylpropane sulfonique) réticulé et neutralisé par de l'ammoniaque, préparé selon le procédé de l'exemple de préparation B de viscosité de l'ordre de 7.000 mPas (cps) dans une solution d'eau à 2% et à 25°C | 1,5 g MA |
| - Glycérine | 5 g |
| - Conservateur qs | |
| - Eau distillée qsp | 100 g |
| pH 5 | |

On obtient une crème gélifiée, légèrement translucide, brillante et homogène.

**<u>EXEMPLE 15</u> :** **Crème hydratante (émulsion huile-dans-eau)**

*Phase grasse*

| | |
|---|---|
| - Polysorbate 60 | 0,8 g |
| - Alcool stéarique | 1,0 g |
| - Glycéryl stéarate (AND) PEG100 stéarate | 1,0 g |
| - Polyisobutène hydrogéné | 10 g |
| - Parfum | 0,3 g |

*Phase aqueuse*

| | |
|---|---|
| - Poly(acide 2-acrylamido 2-méthylpropane sulfonique)réticulé et neutralisé par de l'ammoniaque, préparé selon le procédé de l'exemple de préparation A de viscosité de l'ordre de 16.000 mPas (cps) dans une solution d'eau à 2% et à 25°C | 1,0 g MA |
| - Glycérine | 5,0 g |
| - Conservateur qs | |
| - Eau distillée qs | 100 g |

**<u>ESSAIS COMPARATIFS</u>**

On étudie l'aspect macroscopique de compositions (1) selon l'invention, épaissies par un polymère Poly(acide 2-acrylamido 2-méthylpropane sulfonique) réticulé et neutralisé par de l'ammoniaque, préparé selon le procédé de l'exemple de préparation A de viscosité de l'ordre de 16.000 cps dans une solution d'eau à 2% et à 25°C.

On compare ces compositions à des compositions (2) selon l'art antérieur contenant comme épaississant un

polymère Poly(acide 2-acrylamido 2-méthylpropane sulfonique) non réticulé tel que le produit commercial COSMEDIA HSP1160 vendu par HENKEL.

On fait varier dans chaque type de composition la concentration en polymère épaississant ainsi que le pH de 2 à 7.

Les compositions du type (1) et du type (2) ont comme formulation :

**COMPOSITION (1)**

| | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 mPas (cps) dans une solution d'eau à 2% et à 25°C | **0,5-2 g MA** |
| - Eau distillée qs **pH 2 à 7** | 100 g |

**COMPOSITION (2)**

| | |
|---|---|
| - Poly(acide 2-acrylamido 2-méthylpropane sulfonique) non-réticulé et neutralisé par de la triéthanolamine vendu sous le nom COSMEDIA HSP1160 | **0,5-11 g MA** |
| - Eau distillée qs **pH 2 à 7** | 100 g |

On obtient avec les compositions (1), des gels ou des crèmes, plus ou moins épaissis, stables, homogènes, non-collants et non-filants.

On obtient avec les compositions (2), des gels fluides, instables, hétérogènes, poisseux, filants et collants même à des concentrations élevées en polymère épaississant (11% en matière active).

**Revendications**

1. Utilisation comme agent épaississant et/ou gélifiant dans des compositions cosmétiques et/ou dermatologiques de poly(acide 2-acrylamido 2-méthylpropane sulfonique) réticulés et neutralisés à au moins 90%.

2. Utilisation selon la revendication 1, caractérisée par le fait que les poly(acide 2-acrylamido 2-méthylpropane sulfonique) réticulés et neutralisés à au moins 90% comportent un nombre de motifs de formule générale (1) suivante :

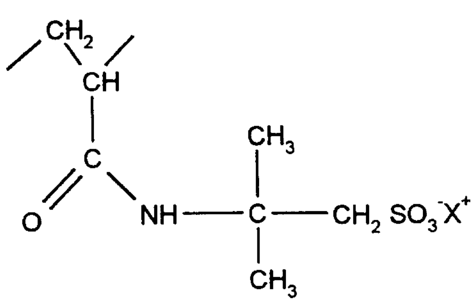

(1)

:

dans une quantité suffisamment élevée pour obtenir des particules de polymère dont le volume hydrodynamique en solution d'eau présente un rayon allant de 10 à 500 nm et dont la distribution est homogène et unimodale.

3. Utilisation selon la revendication 1 ou 2, caractérisée par le fait que les poly(acide 2-acrylamido 2-méthylpropane sulfonique) réticulés et neutralisés à au moins 90% comprennent, distribués de façon aléatoire :

a) de 90 à 99,9% en poids de motifs de formule générale (1) suivante :

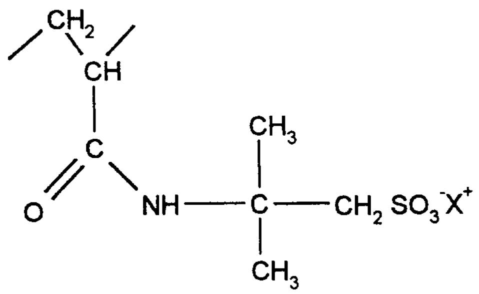

(1)

dans laquelle $X^+$ désigne un cation ou un mélange de cations, au plus 10% mol des cations $X^+$ pouvant être des protons $H^+$;

b) de 0,01 à 10% en poids de motifs réticulants provenant d'au moins un monomère ayant au moins deux doubles-liaisons oléfiniques ; les proportions en poids étant définis par rapport au poids total du polymère.

**4.** Utilisation selon la revendication 3, caractérisée par le fait que les poly(acide 2-acrylamido 2-méthylpropane sulfonique) réticulés et neutralisés à au moins 90% comportent de 98 à 99,5 % en poids de motifs de formule (1) et de 0,2 à 2 % en poids de motifs réticulants.

**5.** Utilisation selon l'une quelconque des revendications 3 à 4, caractérisée par le fait que dans la formule (1) le cation $X^+$ est $NH_4^+$.

**6.** Utilisation selon l'une quelconque des revendications 3 à 5, caractérisée par le fait que les monomères réticulants répondent à la formule générale (2) suivante :

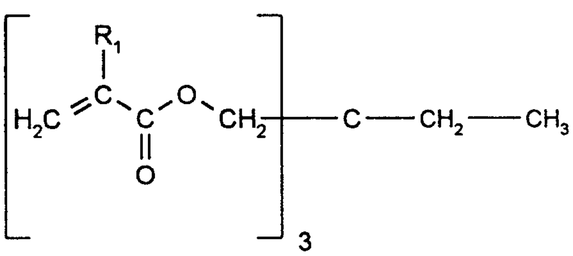

(2)

dans laquelle $R_1$ désigne un atome d'hydrogène ou un alkyle en $C_1$-$C_4$.

**7.** Utilisation selon l'une quelconque des revendications 1 à 6, caractérisée par le fait que le poly(acide 2-acrylamido 2-méthylpropane sulfonique) est réticulé par le triméthylol propane triacrylate.

**8.** Utilisation selon l'une quelconque des revendications 1 à 7, caractérisée par le fait que les polymères de formule (1) présentent une viscosité mesurée au viscosimètre BROOKFIELD, mobile 4, vitesse 100 tours/minute, dans une solution d'eau à 2 % et à 25 °C, supérieure ou égale à 1000 mPas (cps).

**9.** Utilisation selon l'une quelconque des revendications 1 à 7, caractérisée par le fait que les polymères de formule (1) présentent une viscosité mesurée au viscosimètre BROOKFIELD, mobile 4, vitesse 100 tours/minute, dans une solution d'eau à 2 % et à 25°C allant de 5000 à 40000 mPas (cps) et plus particulièrement de 6500 à 35000 mPas (cps).

**10.** Composition cosmétique ou dermatologique, caractérisée par le fait qu'elle contient dans un milieu cosmétiquement acceptable au moins un poly(acide 2-acrylamido 2-méthylpropane sulfonique) réticulé et neutralisé à au moins 90% comprenant une quantité suffisamment élevée de motifs de formule générale (1) suivante :

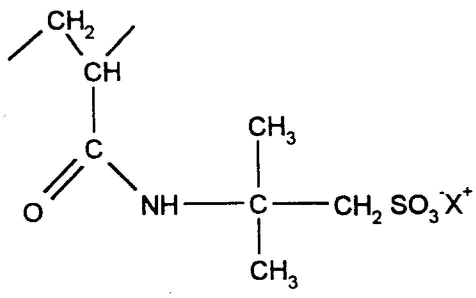

(1)

pour obtenir des particules de polymère dont le volume hydrodynamique en solution d'eau présente un rayon allant de 10 à 500 nm et dont la distribution est homogène et unimodale.

**11.** Composition selon la revendication 10, caractérisée par le fait qu'elle contient dans un milieu cosmétiquement acceptable au moins un poly(acide 2-acrylamido 2-méthylpropane sulfonique) réticulé comprenant, distribués de façon aléatoire :

a) de 90 à 99,9% en poids de motifs de formule générale (1) suivante :

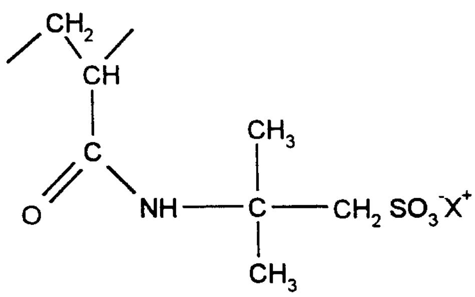

(1)

dans laquelle $X^+$ désigne un cation ou un mélange de cations, au plus 10% mol des cations $X^+$ pouvant être des protons $H^+$;

b) de 0,01 à 10% en poids de motifs réticulants provenant d'au moins un monomère ayant au moins deux doubles-liaisons oléfiniques ; les proportions en poids étant définis par rapport au poids total du polymère.

**12.** Composition selon la revendication 11, caractérisée par le fait que le poly(acide 2-acrylamido 2-méthylpropane sulfonique) réticulé comporte de 98 à 99,5 % en poids de motifs de formule (1) et de 0,2 à 2 % en poids de motifs réticulants.

**13.** Composition selon l'une quelconque des revendications 10 à 12, caractérisée par le fait que dans la formule (1) le cation $X^+$ est $NH_4^+$.

**14.** Composition selon l'une quelconque des revendications 10 à 13, caractérisée par le fait que les monomères réticulants répondent à la formule générale (2) suivante :

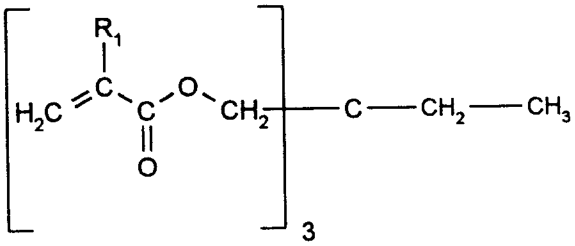 (2)

dans laquelle $R_1$ désigne hydrogène ou un alkyle en $C_1$-$C_4$.

**15.** Composition selon l'une quelconque des revendications 10 à 14, caractérisée par le fait que le poly(acide 2-acrylamido 2-méthylpropane sulfonique) est réticulé par le triméthylol propane triacrylate.

**16.** Composition selon l'une quelconque des revendications 10 à 15, caractérisée par le fait que le poly(acide 2-acrylamido 2-méthylpropane sulfonique) réticulé présente une viscosité mesurée au viscosimètre BROOKFIELD dans une solution d'eau à 2 % et à 25 °C supérieure ou égale à 1000 mPas (cps).

**17.** Composition selon l'une quelconque des revendications 10 à 16, caractérisée par le fait que le poly(acide 2-acrylamido 2-méthylpropane sulfonique) réticulé présente une viscosité mesurée au viscosimètre BROOKFIELD dans une solution d'eau à 2 % et à 25 °C allant de 5000 à 40000 mPas (cps) et plus particulièrement de 6500 à 35000 mPas (cps).

**18.** Composition selon l'une quelconque des revendications 10 à 17, caractérisée par le fait que le poly(acide 2-acrylamido 2-méthylpropane sulfonique) réticulé est présent dans dans des concentrations allant préférentiellement de 0,01 à 20% en poids par rapport au poids total de la composition et plus préférentiellement de 0,1 à 10% en poids.

**19.** Composition selon l'une quelconque des revendications 10 à 18, caractérisée par le fait qu'elle se présente sous forme de dispersion du type lotion ou sérum ; de gel aqueux ou huileux ; d'émulsion de consistance liquide ou semi-liquide du type lait, obtenue par dispersion d'une phase grasse dans une phase aqueuse (huile/eau) ou inversement (eau/huile) ; de suspensions ou d'émulsions de consistance molle, semi-solide ou solide du type crème, gel ; de pain solide.

**20.** Composition selon l'une quelconque des revendications 10 à 19, caractérisée par le fait qu'elle contient un milieu aqueux cosmétiquement et/ou dermatologiquement acceptable.

**21.** Composition selon la revendication 21, caractérisée par le fait qu'elle présente un pH allant de 1 à 13.

**22.** Composition selon l'une quelconque des revendications 10 à 21, caractérisée en ce que le milieu cosmétiquement et/ou dermatologiquement acceptable est constitué d'eau ou d'eau et au moins un solvant organique choisi dans le groupe constitué par les solvants organiques hydrophiles, les solvants organiques lipophiles, les solvants amphiphiles ou leurs mélanges.

**23.** Composition selon la revendication 22, caractérisée en ce que les solvants organiques sont choisis dans le groupe constitué par les alcools mono- ou polyfonctionnels, les polyéthylène glycols éventuellement oxyéthylénés, les esters de propylène glycol, le sorbitol et ses dérivés, les di- alkyles d'isosorbide, les éthers de glycol et les éthers de propylène glycol, les esters gras.

**24.** Composition selon la revendication 22 ou 23, caractérisée en ce que le ou les solvants organiques représentent de 5 % à 98 % du poids total de la composition.

**25.** Composition selon l'une quelconque des revendications 10 à 24, caractérisée en ce qu'elle comprend en plus au moins une phase grasse.

**26.** Composition selon l'une quelconque des revendications 10 à 25, caractérisée en ce que la phase grasse représente de 0 à 50% du poids total de la composition.

27. Composition selon l'une quelconque des revendications 10 à 26, caractérisée par le fait qu'elle contient en plus au moins un additif choisi dans le groupe constitué par les gélifiants et/ou épaississants classiques aqueux ou lipophiles ; des actifs hydrophiles ou lipophiles ; des conservateurs ;des antioxydants ; des parfums ; des émulsionnants ; des agents hydratants ; des émollients ; des sequestrants ; des tensio-actifs ; des polymères ; des agents alcanisants ou acidifiants ; des charges ; des agents anti-radicaux libres ; des céramides ; des filtres solaires ; des répulsifs pour insectes; des agents amincissants ; des matières colorantes ; des bactéricides ; des antipelliculaires.

28. Composition selon l'une quelconque des revendications 10 à 27, caractérisée par le fait qu'elle est utilisée comme produit capillaire rincé ou non-rincé pour le lavage le soin, le conditionnement, le maintien de la coiffure ou la mise en forme des cheveux.

29. Composition selon l'une quelconque des revendications 10 à 27, caractérisée par le fait qu'elle est utilisée comme produit de soin et/ou d'hygiène.

30. Composition selon l'une quelconque des revendications 10 à 27, caractérisée par le fait qu'elle est utilisée comme produit de maquillage.

31. Composition selon l'une quelconque des revendications 10 à 27, caractérisée par le fait qu'elle est utilisée comme produit antisolaire.

32. Composition selon l'une quelconque des revendications 10 à 27, caractérisée par le fait qu'elle est utilisée comme produit de soin bucco-dentaire.

33. Procédé de traitement non-thérapeutique cosmétique de la peau, du cuir chevelu, des cheveux, des cils, des sourcils, des ongles ou des muqueuses, caractérisé par le fait qu'on applique sur le support une composition telle que définie selon l'une quelconque des revendications 10 à 32.

**Claims**

1. Use, as thickener and/or gelling agent in cosmetic and/or dermatological compositions, of crosslinked and at least 90% neutralized poly(2-acrylamido-2-methylpropanesulphonic acids).

2. Use according to Claim 1, characterized in that the crosslinked and at least 90% neutralized poly(2-acrylamido-2-methylpropanesulphonic acids) comprise a number of units of following general formula (1):

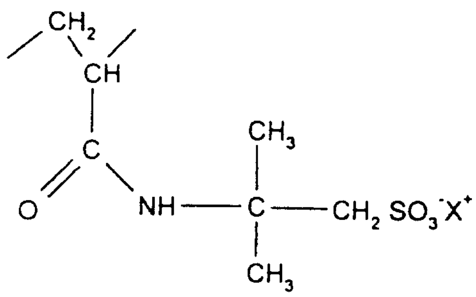

(1)

in an amount which is sufficiently high to obtain polymer particles whose hydrodynamic volume in solution in water has a radius ranging from 10 to 500 nm and whose distribution is homogeneous and unimodal.

3. Use according to Claim 1 or 2, characterized in that the crosslinked and at least 90% neutralized poly(2-acrylamido-2-methylpropanesulphonic acids) comprise, distributed randomly:

a) from 90 to 99.9% by weight of units of general formula (1) below:

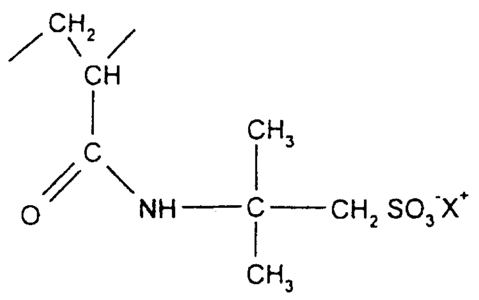

(1)

in which $X^+$ denotes a cation or a mixture of cations, not more than 10 mol% of the cations $X^+$ being able to be protons $H^+$;

b) from 0.01 to 10% by weight of crosslinking units derived from at least one monomer having at least two olefinic double bonds; the weight proportions being defined relative to the total weight of the polymer.

**4.** Use according to Claim 3, characterized in that the crosslinked and at least 90% neutralized poly(2-acrylamido-2-methylpropanesulphonic acids) contain from 98 to 99.5% by weight of units of formula (1) and from 0.2 to 2% by weight of crosslinking units.

**5.** Use according to either one of Claims 3 and 4, characterized in that, in the formula (1), the cation $X^+$ is $NH_4^+$.

**6.** Use according to any one of Claims 3 to 5, characterized in that the crosslinking monomers correspond to the general formula (2) below:

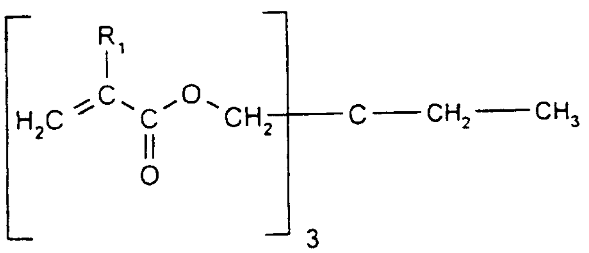

(2)

in which $R_1$ denotes a hydrogen atom or a $C_1$-$C_4$ alkyl.

**7.** Use according to any one of Claims 1 to 6, characterized in that the poly(2-acrylamido-2-methylpropanesulphonic acid) is crosslinked with trimethylolpropane triacrylate.

**8.** Use according to any one of Claims 1 to 7, characterized in that the polymers of formula (1) have a viscosity, measured with a Brookfield viscometer, rotor 4, speed 100 revolutions/minute, in an aqueous 2% solution and at 25°C, of greater than or equal to 1000 mPa • s (cps).

**9.** Use according to any one of Claims 1 to 7, characterized in that the polymers of formula (1) have a viscosity, measured with a Brookfield viscometer, rotor 4, speed 100 revolutions/minute, in an aqueous 2% solution and at 25°C, ranging from 5000 to 40,000 mPa • s (cps) and more particularly from 6500 to 35,000 mPa • s (cps).

**10.** Cosmetic or dermatological composition, characterized in that it contains, in a cosmetically acceptable medium, at least one crosslinked and at least 90% neutralized poly(2-acrylamido-2-methylpropanesulphonic acid) comprising an amount which is sufficiently high of units of following general formula (1):

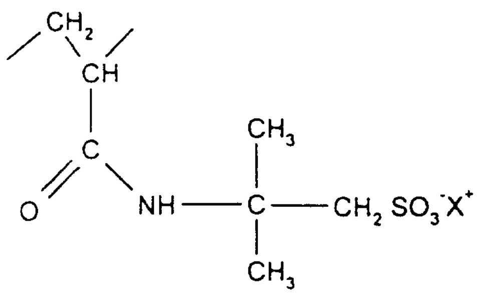

(1)

to obtain polymer particles whose hydrodynamic volume in solution in water has a radius ranging from 10 to 500 nm and whose distribution is homogeneous and unimodal.

**11.** Composition according to Claim 10, characterized in that it contains, in a cosmetically acceptable medium, at least one crosslinked poly (2-acrylamido-2-methylpropanesulphonic acid) comprising, distributed randomly:

a) from 90 to 99.9% by weight of units of general formula (1) below:

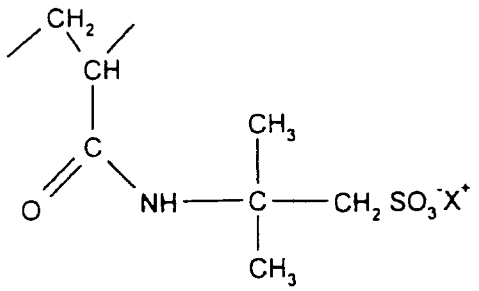

(1)

in which $X^+$ denotes a cation or a mixture of cations, not more than 10 mol% of the cations $X^+$ being able to be protons $H^+$;

b) from 0.01 to 10% by weight of crosslinking units derived from at least one monomer having at least two olefinic double bonds; the weight proportions being defined relative to the total weight of the polymer.

**12.** Composition according to Claim 11, characterized in that the crosslinked poly(2-acrylamido-2-methylpropanesulphonic acid) contains from 98 to 99.5% by weight of units of formula (1) and from 0.2 to 2% by weight of crosslinking units.

**13.** Composition according to any one of Claims 10 to 12, characterized in that, in the formula (1), the cation $X^+$ is $NH_4^+$.

**14.** Composition according to any one of Claims 10 to 13, characterized in that the crosslinking monomers correspond to the general formula (2) below:

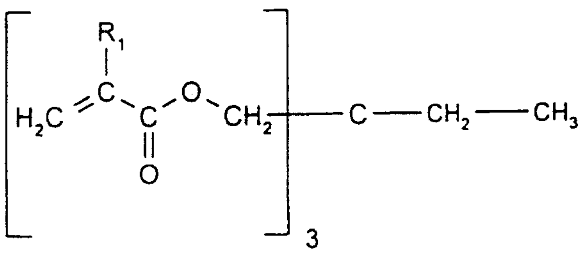

(2)

in which $R_1$ denotes hydrogen or a $C_1$-$C_4$ alkyl.

**15.** Composition according to any one of Claims 10 to 14, characterized in that the poly(2-acrylamido-2-methylpropanesulphonic acid) is crosslinked with trimethylolpropane triacrylate.

**16.** Composition according to any one of Claims 10 to 15, characterized in that the crosslinked poly(2-acrylamido-2-methylpropanesulphonic acid) has a viscosity, measured with a Brookfield viscometer in an aqueous 2% solution and at 25°C, of greater than or equal to 1000 mPa • s (cps).

**17.** Composition according to any one of Claims 10 to 16, characterized in that the crosslinked poly(2-acrylamido-2-methylpropanesulphonic acid) has a viscosity, measured with a Brookfield viscometer in an aqueous 2% solution and at 25°C, ranging from 5000 to 40,000 mPa • s (cps) and more particularly from 6500 to 35,000 mPa • s (cps).

**18.** Composition according to any one of Claims 10 to 17, characterized in that the crosslinked poly(2-acrylamido-2-methylpropanesulphonic acid) is present in concentrations preferably ranging from 0.01 to 20% by weight relative to the total weight of the composition and more preferably from 0.1 to 10% by weight.

**19.** Composition according to any one of Claims 10 to 18, characterized in that it is in the form of a dispersion of the lotion or serum type; aqueous or oily gel; emulsion of liquid or semi-liquid consistency of the milk type, obtained by dispersing a fatty phase in an aqueous phase (oil/water) or, conversely, (water/oil); suspensions or emulsions of soft, semi-solid or solid consistency of the cream or gel type; solid bar.

**20.** Composition according to any one of Claims 10 to 19, characterized in that it contains a cosmetically and/or dermatologically acceptable aqueous medium.

**21.** Composition according to Claim 20, characterized in that it has a pH ranging from 1 to 13.

**22.** Composition according to any one of Claims 10 to 21, characterized in that the cosmetically and/or dermatologically acceptable medium consists of water or of water and at least one organic solvent chosen from the group consisting of hydrophilic organic solvents, lipophilic organic solvents, amphiphilic solvents or mixtures thereof.

**23.** Composition according to Claim 22, characterized in that the organic solvents are chosen from the group consisting of mono- or polyfunctional alcohols, optionally oxyethylenated polyethylene glycols, propylene glycol esters, sorbitol and derivatives thereof, dialkyl isosorbides, glycol ethers and propylene glycol ethers, and fatty esters.

**24.** Composition according to Claim 22 or 23, characterized in that the organic solvent or solvents represent from 5% to 98% of the total weight of the composition.

**25.** Composition according to any one of Claims 10 to 24, characterized in that it also comprises at least one fatty phase.

**26.** Composition according to any one of Claims 10 to 25, characterized in that the fatty phase represents from 0 to 50% of the total weight of the composition.

**27.** Composition according to any one of Claims 10 to 26, characterized in that it also contains at least one additive chosen from the group consisting of standard aqueous or lipophilic gelling agents and/or thickeners; hydrophilic or lipophilic active agents; preserving agents; antioxidants; fragrances; emulsifiers; moisturizers; emollients; sequestering agents; surfactants; polymers; basifying or acidifying agents; fillers; anti-free-radical agents; ceramides; sunscreens; insect repellents; slimming agents, dyestuffs; bactericides; antidandruff agents.

**28.** Composition according to any one of Claims 10 to 27, characterized in that it is used as a rinse-out or leave-in hair product for washing, care, conditioning, maintenance of the style or shaping of the hair.

**29.** Composition according to any one of Claims 10 to 27, characterized in that it is used as a care and/or hygiene product.

**30.** Composition according to any one of Claims 10 to 27, characterized in that it is used as a make-up product.

31. Composition according to any one of Claims 10 to 27, characterized in that it is used as an antisun product.

32. Composition according to any one of Claims 10 to 27, characterized in that it is used as a buccodental care product.

33. Process for the non-therapeutic cosmetic treatment of the skin, the scalp, the hair, the eyelashes, the eyebrows, the nails or the mucous membranes, characterized in that a composition as defined according to any one of Claims 10 to 32 is applied to the support.

**Patentansprüche**

1. Verwendung von vernetzten und zu mindestens 90 % neutralisierten Poly(2-acrylamido-2-methylpropansulfonsäuren) als Verdickungsmittel und/oder Gelbildner in kosmetischen und/oder dermatologischen Zusammensetzungen.

2. 2. Verwendung nach Anspruch 1, dadurch gekennzeichnet, daß die vernetzten und zu mindestens 90 % neutralisierten Poly(2-acrylamido-2-methylpropansulfonsäuren) Einheiten der folgenden allgemeinen Formel (1)

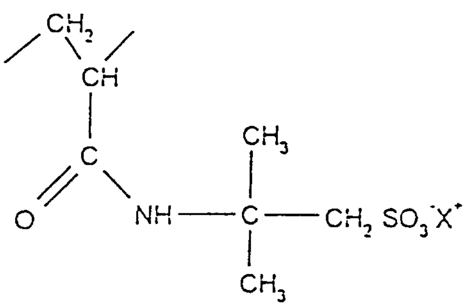

(1)

in einer Anzahl enthalten, die ausreichend groß ist, um Polymerpartikel zu erhalten, deren hydrodynamisches Volumen in wäßriger Lösung einen Radius von 10 bis 500 nm aufweist und deren Verteilung homogen und unimodal ist.

3. Verwendung nach Anspruch 1 oder 2, dadurch gekennzeichnet, daß die vernetzten und zu mindestens 90 % neutralisierten Poly(2-acrylamido-2-methylpropansulfonsäuren) statistisch verteilt enthalten:

a) 90 bis 99,9 Gew.-% Einheiten der folgenden allgemeinen Formel (1):

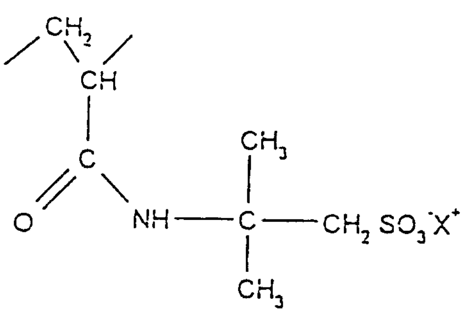

(1),

in der $X^+$ ein Kation oder Kationengemisch bedeutet, wobei höchstens 10 Mol-% der Kationen $X^+$ Protonen $H^+$ sein können,
b) 0,01 bis 10 Gew.-% vernetzende Einheiten, die von mindestens einem Monomer stammen, das mindestens zwei olefinische Doppelbindungen aufweist, wobei die Gewichtsanteile bezogen auf das Gesamtgewicht des Polymers definiert sind.

4. Verwendung nach Anspruch 3, dadurch gekennzeichnet, daß die vernetzten und zu mindestens 90 % neutralisierten Poly(2-acrylamido-2-methylpropansulfonsäuren) 98 bis 99,5 Gew.-% Einheiten der Formel (1) und 0,2 bis 2 Gew.-% vernetztende Einheiten enthalten.

**5.** Verwendung nach einem der Ansprüche 3 und 4, dadurch gekennzeichnet, daß in der Formel (1) das Kation $X^+$ $NH_4^+$ ist.

**6.** Verwendung nach einem der Ansprüche 3 bis 5, dadurch gekennzeichnet, daß die vernetzenden Monomere der folgenden allgemeinen Formel (2)

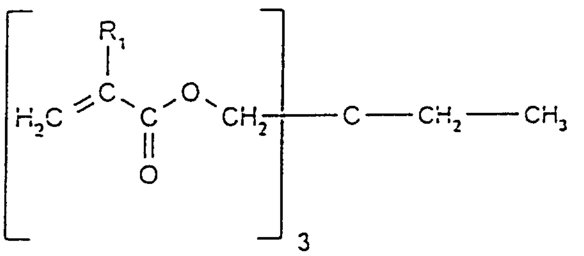

(2)

entsprechen, in der $R_1$ ein Wasserstoffatom oder ein $C_{1-4}$-Alkyl bedeutet.

**7.** Verwendung nach einem der Ansprüche 1 bis 6, dadurch gekennzeichnet, daß die Poly(2-acrylamido-2-methylpropansulfonsäure) mit Trimethylolpropantriacrylat vernetzt ist.

**8.** Verwendung nach einem der Ansprüche 1 bis 7, dadurch gekennzeichnet, daß die Polymere der Formel (1) eine mit einem BROOKFIELD-Viskosimeter, Drehkörper 4, Geschwindigkeit 100 U/min bei 25 °C in 2%iger wäßriger Lösung gemessene Viskosität von 1 000 mPas (cP) oder darüber aufweisen.

**9.** Verwendung nach einem der Ansprüche 1 bis 7, dadurch gekennzeichnet, daß die Polymere der Formel (1) eine mit einem BROOKFIELD-Viskosimeter, Drehkörper 4, Geschwindigkeit 100 U/min bei 25 °C in 2%iger wäßriger Lösung gemessene Viskosität von 5 000 bis 40 000 mPas (cP) und insbesondere 6 500 bis 35 000 mPas (cP) aufweisen.

**10.** Kosmetische oder dermatologische Zusammensetzung, dadurch gekennzeichnet, daß sie in einem kosmetisch akzeptablen Medium mindestens eine vernetzte und zu mindestens 90 % neutralisierte Poly(2-acrylamido-2-methylpropansulfonsäure) enthält, die Einheiten der folgenden allgemeinen Formel (1)

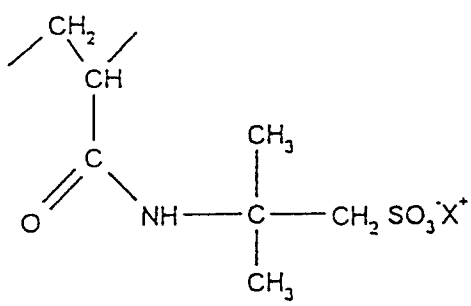

(1)

in einer Anzahl enthält, die ausreichend groß ist, um Polymerpartikel zu erhalten, deren hydrodynamisches Volumen in wäßriger Lösung einen Radius von 10 bis 500 nm aufweist und deren Verteilung homogen und unimodal ist.

**11.** Zusammensetzung nach Anspruch 10, dadurch gekennzeichnet, daß sie in einem kosmetisch akzeptablen Medium mindestens eine vernetzte Poly(2-acrylamido-2-methylpropansulfonsäure) enthält, die statistisch verteilt enthält:

a) 90 bis 99,9 Gew.-% Einheiten der folgenden allgemeinen Formel (1)

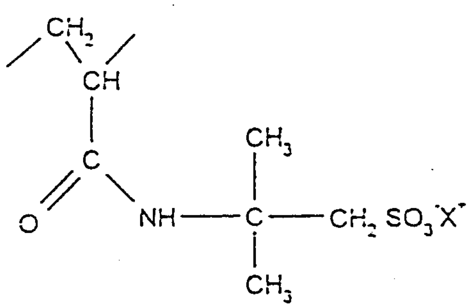

(1),

in der $X^+$ ein Kation oder ein Kationengemisch bedeutet, wobei höchstens 10 Mol-% der Kationen $X^+$ Protonen $H^+$ sein können;

b) 0,01 bis 10 Gew.-% vernetzende Einheiten, die von mindestens einem Monomer stammen, das mindestens zwei olefinische Doppelbindungen aufweist, wobei die Gewichtsanteile bezogen auf das Gesamtgewicht des Polymers definiert sind.

**12.** Zusammensetzung nach Anspruch 11, dadurch gekennzeichnet, daß die vernetzte Poly(2-acrylamido-2-methylpropansulfonsäure) 98 bis 99,5 Gew.-% Einheiten der Formel (1) und 0,2 bis 2 Gew.-% vernetzende Einheiten enthält.

**13.** Zusammensetzung nach einem der Ansprüche 10 bis 12, dadurch gekennzeichnet, daß in der Formel (1) das Kation $X^+$ $NH_4^+$ ist.

**14.** Zusammensetzung nach einem der Ansprüche 10 bis 13, dadurch gekennzeichnet, daß die vernetzenden Monomere die folgende allgemeine Formel (2)

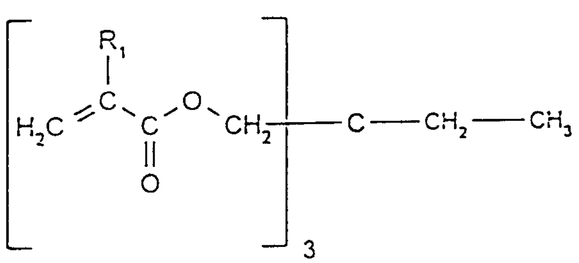

(2)

aufweisen, in der $R_1$ Wasserstoff oder ein $C_{1-4}$-Alkyl bedeutet.

**15.** Zusammensetzung nach einem der Ansprüche 10 bis 14, dadurch gekennzeichnet, daß die Poly(2-acrylamido-2-methylpropansulfonsäure) mit Trimethylolpropantriacrylat vernetzt ist.

**16.** Zusammensetzung nach einem der Ansprüche 10 bis 15, dadurch gekennzeichnet, daß die vernetzte Poly(2-acrylamido-2-methylpropansulfonsäure) eine mit einem BROOKFIELD-Viskosimeter bei 25 °C in 2%iger wäßriger Lösung gemessene Viskosität von 1 000 mPas (cP) oder darüber aufweist.

**17.** Zusammensetzung nach einem der Ansprüche 10 bis 16, dadurch gekennzeichnet, daß die vernetzte Poly(2-acrylamido-2-methylpropansulfonsäure) eine mit einem BROOKFIELD-Viskosimeter bei 25 °C in 2%iger wäßriger Lösung gemessene Viskosität von 5 000 bis 40 000 mPas (cP) und insbesondere 6 500 bis 35 000 mPas (cP) aufweist.

**18.** Zusammensetzung nach einem der Ansprüche 10 bis 17, dadurch gekennzeichnet, daß die vernetzte Poly(2-acrylamido-2-methylpropansulfonsäure) in Konzentrationen von vorzugsweise 0,01 bis 20 Gew.-%, bezogen auf das Gesamtgewicht der Zusammensetzung, und noch bevorzugter 0,1 bis 10 Gew.-% enthalten ist.

**19.** Zusammensetzung nach einem der Ansprüche 10 bis 18, dadurch gekennzeichnet, daß sie in Form einer Dispersion vom Typ der Lotionen oder Sera, in Form eines wäßrigen oder öligen Gels, einer Emulsion mit flüssiger oder halbflüssiger Konsistenz vom Milch-Typ, die durch Dispergieren einer Fettphase in einer wäßrigen Phase (Öl/Wasser) oder umgekehrt (Wasser/Öl) erhalten wird, in Form von Suspensionen oder Emulsionen mit weicher, halbfester oder fester Konsistenz vom Typ der Cremes, Gele, in Form eines festen Stücks vorliegt.

**20.** Zusammensetzung nach einem der Ansprüche 10 bis 19, dadurch gekennzeichnet, daß sie ein kosmetisch und/oder dermatologisch akzeptables wäßriges Medium enthält.

**21.** Zusammensetzung nach einem der Ansprüche 10 bis 20, dadurch gekennzeichnet, daß sie einen pH-Wert von 1 bis 13 aufweist.

**22.** Zusammensetzung nach einem der Ansprüche 10 bis 21, dadurch gekennzeichnet, daß das kosmetisch und/oder dermatologisch akzeptable Medium aus Wasser oder aus Wasser und mindestens einem organischen Lösungsmittel besteht, das unter hydrophilen organischen Lösungsmitteln, lipophilen organischen Lösungsmitteln, amphiphilen Lösungsmitteln oder deren Gemischen ausgewählt ist.

**23.** Zusammensetzung nach Anspruch 22, dadurch gekennzeichnet, daß die organischen Lösungsmittel unter einwertigen und mehrwertigen Alkoholen, gegebenenfalls ethoxylierten Polyethylenglykolen, Propylenglykolestern, Sorbit und dessen Derivaten, Dialkyldiisosorbiden, Glykolethern und Propylenglykolethern, Fettestern ausgewählt sind.

**24.** Zusammensetzung nach Anspruch 22 oder 23, dadurch gekennzeichnet, daß das oder die organischen Lösungsmittel 5 bis 98 % des Gesamtgewichts der Zusammensetzung ausmachen.

**25.** Zusammensetzung nach einem der Ansprüche 10 bis 24, dadurch gekennzeichnet, daß sie außerdem mindestens eine Fettphase enthält.

**26.** Zusammensetzung nach einem der Ansprüche 10 bis 25, dadurch gekennzeichnet, daß die Fettphase 0 bis 50 % des Gesamtgewichts der Zusammensetzung ausmacht.

**27.** Zusammensetzung nach einem der Ansprüche 10 bis 26, dadurch gekennzeichnet, daß sie außerdem mindestens einen Zusatz enthält, der unter herkömmlichen wäßrigen oder lipophilen Gelbildnern und/oder Verdickungsmitteln, hydrophilen oder lipophilen Wirkstoffen, Konservierungsmitteln, Antioxidantien, Parfums, Emulgatoren, Hydratisierungsmitteln, weichmachenden Mitteln, Maskierungsmitteln, grenzflächenaktiven Stoffen, Polymeren, Alkalisierungs- oder Säuerungsmitteln, Füllstoffen, Mitteln gegen freie Radikale, Ceramiden, Lichtschutzfiltern, Insektenrepellents, schlanker machenden Mitteln, Färbemitteln, Bakteriziden, Antischuppenmitteln ausgewählt ist.

**28.** Zusammensetzung nach einem der Ansprüche 10 bis 27, dadurch gekennzeichnet, daß sie als auszuspülendes oder nicht auszuspülendes Haarbehandlungsprodukt für die Haarwäsche, zur Pflege, zur Konditionierung, zur Erhaltung der Frisur oder für die Gestaltung der Haare verwendet wird.

**29.** Zusammensetzung nach einem der Ansprüche 10 bis 27, dadurch gekennzeichnet, daß sie als Pflegeprodukt und/oder Hygieneprodukt verwendet wird.

**30.** Zusammensetzung nach einem der Ansprüche 10 bis 27, dadurch gekennzeichnet, daß sie als Schminkprodukt verwendet wird.

**31.** Zusammensetzung nach einem der Ansprüche 10 bis 27, dadurch gekennzeichnet, daß sie als Lichtschutzprodukt verwendet wird.

**32.** Zusammensetzung nach einem der Ansprüche 10 bis 27, dadurch gekennzeichnet, daß sie als Produkt für die buckaldentale Pflege verwendet wird.

**33.** Verfahren zur nicht-therapeutischen kosmetischen Behandlung der Haut, der Kopfhaut, der Haare, der Wimpern, der Augenbrauen, der Nägel oder der Schleimhäute, dadurch gekennzeichnet, daß auf den Träger eine wie in einem der Ansprüche 10 bis 32 definierte Zusammensetzung aufgetragen wird.